# EUROPÄISCHE PATENTANMELDUNG

(11) **EP 4 843 590 A1**
(43) Veröffentlichungstag der Anmeldung: **25.11.2026**
(21) Anmeldenummer: 25177368.5
(22) Anmeldetag: 19.05.2025
(51) Int. Cl.: A61M 5/142

(54) **INFUSIONSPUMPE MIT FILTERAUFNAHME UND INFUSIONSLEITUNGSSYSTEM MIT FILTER**

(71) Anmelder: B. Braun Melsungen AG, 34212 Melsungen (DE)
(72) Erfinder: DISCHER, Markus, 34593 Knüllwald (DE); UMBACH, Alina, 34560 Fritzlar (DE)
(74) Vertreter: Winter, Brandl - Partnerschaft mbB

(57) **Zusammenfassung**

Offenbart sind eine Infusionspumpe (20) und ein Infusionsleitungssystem (2; 102) für eine Infusionspumpe (20) und ein Infusionssystem mit einer Infusionspumpe (20) und mit einem Infusionsleitungssystem (2; 102). In dem Infusionsleitungssystem (2; 102) ist ein Filter (1) zwischengeschaltet bzw. eingesetzt, der eine Filtermembran (8) aufweist. Dieser Filter (1) ist an einer Stelle in dem Infusionsleitungssystem (2; 102) angeordnet, die nach dessen Einsetzen in die Infusionspumpe (20) direkt stromab von einem Pumpenabschnitt und im Falle eines Luftsensors (30) der Infusionspumpe (20) stromauf von diesem angeordnet ist.

## Beschreibung

### Technisches Gebiet

Die vorliegende Offenbarung betrifft eine medizinische Infusionspumpe und ein Infusionsleitungssystem (Überleitungssystem) für eine Infusionspumpe und ein Infusionssystem mit einer Infusionspumpe und einem Infusionsleitungssystem.

Die Dosierung medizinischer Infusionsflüssigkeiten und deren Zuführung zu einem Blutgefäß eines Patienten kann über eine Infusionspumpe erfolgen. Ein mögliches Wirkprinzip einer solchen Infusionspumpe ist, einem Infusionsleitungssystem, durch das die Infusionsflüssigkeit geleitet wird, mittels einer Verdrängereinheit der Infusionspumpe eine peristaltische Bewegung aufzuprägen, wodurch die Infusionsflüssigkeit von einem Infusionsflüssigkeitsbehälter hin zu einem Zugang am Patienten gefördert wird.

### Hintergrund der Offenbarung

Bei Infusionspumpen sollte vermieden werden, dass Luft und Partikel dem Patienten zugeführt werden. Luft kann z.B. an Kupplungen oder an Ventilen des Infusionsleitungssystems in die Infusionsflüssigkeit eintreten. Insbesondere soll auch vermieden werden, dass Luftblasen zum Patienten gefördert werden, die durch das Einwirken der Fördermittels der Infusionspumpe, wie z.B. einer peristaltischen Verdrängereinheit oder Schlauchrollen in der Infusionsflüssigkeit auftreten können.

### Stand der Technik

Aus hausinternem Stand der Technik ist ein Infusionsleitungssystem für Schwerkraftinfusion bekannt, das an seinem vom Patienten abgewandten Bereich eine Tropfkammer hat, die als Luftabscheider dient. Zusätzlich ist an einem in Schwerkraftrichtung unteren Bereich der Tropfkammer eine hydrophile (Fluid-) Filtermembran eingelegt, die als ein Set/Satz von hydrophilen Kapillaren ausgebildet ist, welche als eine Vielzahl an zusammengepackten, parallelen Röhrchen angesehen werden können. Wenn nun eine Infusionsflüssigkeit durch die Filtermembran strömt, strömt diese durch die parallelen Kapillaren. Damit wird vermieden, dass Luft von der betroffenen Stelle in das (stromab angeordnete) Infusionsleitungssystem gelangt.

Oftmals sind die Infusionsleitungssysteme als Einwegprodukte ausgelegt, die nach Beendigung der Infusion und/oder nach dem Leerlaufen eines stromauf(wärts) daran angeschlossenen Infusionsflüssigkeitsbehälters aus der Infusionspumpe entfernt und weggeworfen werden.

In der WO 2022/233731 A1 der Anmelderin ist eine vergleichbare Filtermembran offenbart. Diese ist in einem extra Filtergehäuse aufgenommen, das einen Zulauf und einen Ablauf für die Infusionsflüssigkeit hat und ansonsten geschlossen ist. Der Zu- und der Ablauf werden an Schlauchsegmente des übergeordneten Infusionsleitungssystems angeschlossen. Im Inneren des Filtergehäuses, d.h. im Bereich einer Kavität (Hohlraum), sind die Filtermembran und ein Entlüftungs-/Belüftungsfilter angeordnet. Dabei dient die Filtermembran als Luftbarriere, und der Belüftungsfilter ermöglicht den Transport von Luft, welche sich im Bereich der Kavität sammelt, zu einer Außenseite des Filtergehäuses.

Eine peristaltische Infusionspumpe linearer Bauart ist in der Druckschrift EP 0 484 717 A1 der Anmelderin gezeigt. Sie hat eine Schlauchaufnahme für einen Abschnitt eines Infusionsleitungssystems, durch welches die Infusionsflüssigkeit gefördert wird, sowie eine Verdrängereinheit mit einer Vielzahl von schieberartigen Verdrängern, die in der Schlauchaufnahme nebeneinander angeordnet sind und quer zur Schlauchaufnahme linearbeweglich in einem schachtartigen Gehäuseabschnitt geführt sind. Nachdem der Abschnitt des Infusionsleitungssystems in die Schlauchaufnahme eingesetzt ist, wird eine Frontklappe der Infusionspumpe geschlossen, sodass der Abschnitt des Infusionsleitungssystems in der Schlauchaufnahme festgelegt ist.

Nachteilig an derartigen Infusionspumpen bzw. an derartigen Infusionsleitungssystemen für Infusionspumpen bzw. an derartigen (aus beiden Komponenten bestehenden) Infusionssystemen ist, dass die Luftabscheidung nicht optimal ist. Im Falle eines (z.B. ebenfalls hinter der Frontklappe angeordneten) Luftsensors der Infusionspumpe werden Luftblasen erkannt und entsprechende Alarme ausgelöst. Diese Alarme sind für den Patienten von Nachteil, weil die Infusion gestoppt wird, und sie sind für das Bedienpersonal von Nachteil, weil sie jedes Mal einen manuellen Eingriff erfordern.

Weiterhin ist nachteilig, dass ein erheblicher Rest der Infusionsflüssigkeit stromab des Luftabscheiders in dem Infusionsleitungssystem verbleibt.

Insbesondere ist bei den Infusionspumpen und bei den Infusionsschlauchsystemen des Standes der Technik nachteilig, dass während des Pumpens durch das Fördermittel, z.B. durch die peristaltische Verdrängereinheit, Luftblasen gebildet oder in die Infusionsflüssigkeit eingebracht werden.

### Kurzbeschreibung der Offenbarung

Aufgabe der vorliegenden Offenbarung ist es, eine Infusionspumpe und ein Infusionsleitungssystem (Überleitungssystem) für eine (Anwendung an einer) Infusionspumpe und ein (aus beiden Komponenten bestehendes) Infusionssystem zu schaffen, bei denen die Luftabscheidung der durch ein Fördermittel, z.B. eine peristaltische Verdrängereinheit, gebildeten oder eingebrachten Luftblasen verbessert ist.

Diese Aufgabe wird im Hinblick auf die Infusionspumpe mit der Merkmalskombination des Anspruchs 1 und im Hinblick auf das Infusionsleitungssystem mit der Merkmalskombination des Anspruchs 8 und im Hinblick auf das Infusionssystem mit der Merkmalskombination des Anspruchs 14 gelöst.

Die Infusionspumpe gemäß der Offenbarung hat eine Schlauchaufnahme zum teilweisen Einlegen eines Infusionsleitungssystems, wobei in der Schlauchaufnahme ein Fördermittel angeordnet ist, das z.B. eine peristaltische Verdrängereinheit sein kann. Offenbarungsgemäß ist (vorzugsweise ohne Zwischenschaltung weiterer Komponenten) stromabwärts des Fördermittels eine Filteraufnahme mit einem Filteraufnahmeraum für ein Filtergehäuse oder eines Filtergehäuses vorgesehen.

Damit ist die Luftabscheidung gegenüber dem Stand der Technik stromab(wärts) hinter kritische Stellen wie Ventile oder Kupplungen oder Y-Stücke oder Hähne des eingesetzten Infusionsleitungssystems verschoben worden und scheidet somit auch dort eingetretene Luftbläschen ab. Insbesondere ist gemäß der offenbarungsgemäßen Infusionspumpe die Abscheidung der durch das Fördermittel, z.B. die peristaltische Verdrängereinheit, gebildeten oder eingebrachten Luftblasen ermöglicht oder zumindest verbessert.

Bei einer besonders bevorzugten Weiterbindung der Infusionspumpe ist stromab(wärts) der Filteraufnahme ein Luftsensor der Schlauchaufnahme angeordnet, mit dem Luft (und vorzugsweise Partikel) in dem Infusionsleitungssystem erkennbar ist bzw. sind. Entsprechend ist die Filteraufnahme bevorzugt zwischen der Verdrängereinheit und dem Luftsensor vorgesehen.

Damit ist eine Infusionspumpe geschaffen, bei der die Luftabscheidung verbessert ist, so dass die Häufigkeit von Alarmen verringert ist, weil die Luftabscheidung an der Filteraufnahme stromauf(wärts) des Luftsensors und dabei am eingesetzten Infusionsleitungssystem soweit wie möglich stromab(wärts) erfolgt.

Die Infusionspumpe kann offenbarungsgemäß auch ohne Luftsensor ausgebildet sein. Auch in diesem Fall findet die Luftabscheidung in dem Filter stromabwärts vom Fördermittel und möglichst direkt hinter dem Fördermittel statt.

Unter "Filter" ist in dieser Schrift ein Luftabscheider zu verstehen, der eine Filtermembran umfasst. Eine derartige Filtermembran kann auch Partikel zurückhalten.

Insbesondere wenn das eingelegte Infusionsleitungssystem ein Einwegartikel ist und daher die in die Filteraufnahme der offenbarungsgemäßen Infusionspumpe eingesetzten Filter entsprechend oft eingesetzt und wieder entnommen werden müssen, ist es von Vorteil, wenn die Filteraufnahme eine formschlüssige Verbindung, insbesondere eine Clips- oder Schnappverbindung (z.B. Rasthaken) für das Filtergehäuse aufweist.

Bei einer besonders bevorzugten Weiterbildung hat das Gehäuse der Infusionspumpe einen Hauptkörper, an den eine Frontklappe oder ein anderer Schlauchfachdeckel angelenkt ist, durch die oder den die Schlauchaufnahme zumindest abschnittsweise abdeckbar ist. Insbesondere sind der Luftsensor und/oder die Verdrängereinheit abdeckbar. Offenbarungsgemäß ist der Filteraufnahmeraum durch die geschlossene Frontklappe oder den geschlossenen Schlauchfachdeckel abgedeckt und begrenzt. Vorzugsweise ist damit das Filtergehäuse in dem Filteraufnahmeraum festlegbar.

Vorzugsweise ist der Filteraufnahmeraum von einer Aussparung oder Mulde begrenzt oder gebildet, die in der Frontklappe bzw. im Schlauchfachdeckel oder im Hauptkörper gebildet sein kann. Damit ist die korrekte Positionierung des Filtergehäuses in der Filteraufnahme für das Bedienpersonal deutlich erkennbar.

Wenn die Aussparung oder Mulde eine konkave Form (Negativform) hat, die zumindest abschnittsweise an eine konvexe Form (Positivform) des Filtergehäuses angepasst ist, ist eine Positionierhilfe geschaffen. Diese kann als Basis für die Positionierung z.B. von Kupplungsstücken bzw. zumindest eines Kupplungsstücks eines Pumpensegments des Infusionsleitungssystems in entsprechenden Aufnahmemulden bzw. in einer entsprechenden Aufnahmemulde der Schlauchaufnahme dienen.

Bei einer besonders betriebssicheren Weiterbildung der Infusionspumpe ist im Bereich des Filteraufnahmeraumes ein Sensor vorgesehen, mit dem erkannt werden kann, ob ein Filtergehäuse (und damit ein Infusionsleitungssystem) passend eingesetzt ist.

Vorzugsweise hat der vorgesehene Luftsensor beidseitig einer Mittelachse angeordnete Sensorik. Vorzugsweise hat die Verdrängereinheit eine Vielzahl von Verdrängern, die radial zur Mittelachse bewegbar sind.

Wenn die Schlauchaufnahme eine derartige vorzugsweise durchgehende Mittelachse hat, entlang der das (gerade ausgerichtete) Infusionsleitungssystem teilweise einlegbar ist, dann ist es bevorzugt, wenn der Filteraufnahmeraum von der Mittelachse durchdrungen ist.

Eine Weiterbildung der Infusionspumpe weist ein Klemmenmodul zur Aufnahme einer als Schiebeklemme ausgebildeten Schlauchklemme auf. Dann weist das Klemmenmodul besonders bevorzugt einen Photodetektor zur Erkennung der Farbe der Schiebeklemme auf. Mit dieser Farbe ist einer von verschiedenen medizinischen Fachbereichen bzw. Anwendungsbereichen des eingelegten Infusionsleitungssystems definiert, an dem die Schiebeklemme unverlierbar befestigt ist. Die Infusionspumpe kann derart eingerichtet sein, dass sie auf Grund des über die Farbe erkannten Anwendungsbereiches eine Voreinstellung vornimmt und z.B. auf einem Display passende Funktionen anbietet.

Die Infusionspumpe kann zur Ausgabe eines Unterdruckalarms ausgebildet und eingerichtet sein, der bei Leerlauf des Infusionsleitungssystems ausgegeben wird.

Das offenbarungsgemäße Infusionsleitungssystem ist zum teilweisen Einlegen in eine offenbarungsgemäße Infusionspumpe eingerichtet und ausgelegt und weist einen Filter mit einem Filtergehäuse auf. Das Filtergehäuse ist über einen Einlass mit einem behälterseitigen Infusionsschlauch und über einen Auslass mit einem patientenseitigen Infusionsschlauch verbunden. Im Innern des Filtergehäuses ist eine vorzugsweise hydrophile (Fluid-) Filtermembran angeordnet, die vorzugsweise eine Vielzahl von hydrophilen Kapillaren (Röhrchen) aufweist. An dem behälterseitigen Infusionsschlauch stromauf des Filters ist (vorzugsweise ohne Zwischenschaltung weiterer Komponenten) ein Pumpenabschnitt angeordnet oder gebildet, der in Wirkverbindung mit dem Fördermittel, vorzugsweise der peristaltischen Verdrängereinheit der Infusionspumpe bringbar ist. Wenn nun eine Infusionsflüssigkeit durch die Filtermembran strömt, wird vermieden, dass Luft und Partikel von der betroffenen Stelle in den patientenseitigen Infusionsschlauch und damit zum Patienten gelangen.

Damit ist ein Infusionsleitungssystem geschaffen, bei der die Luftabscheidung verbessert ist, weil die Luftabscheidung soweit stromab wie möglich erfolgt und damit alle an Ventilen oder Kupplungen oder Hähnen eventuell eingetretenen Luftbläschen abscheidet. Weiterhin ist die Restmenge von Infusionsflüssigkeit, die nach dem Leerlaufen des Infusionsflüssigkeitsbehälters im Infusionsleitungssystem verbleibt, verringert. Mit dem offenbarungsgemäßen Infusionsleitungssystem ist insbesondere die Abscheidung der durch das Fördermittel, z.B. durch die peristaltische Verdrängereinheit, gebildeten oder eingebrachten Luftblasen ermöglicht oder zumindest verbessert.

Bei einer besonders bevorzugten Weiterbildung des Infusionsleitungssystems ist an dem patientenseitigen Infusionsschlauch direkt stromab des Filters ein Sensorabschnitt angeordnet oder gebildet, der in Wirkverbindung mit einem Luftsensor der Infusionspumpe bringbar ist.

Wenn die Filtermembran aus einer Vielzahl von Kapillaren besteht, die vorzugsweise senkrecht zur Durchflussrichtung angeordnet sind, entsteht eine Haltekraft an der Infusionsflüssigkeit, die ein Nachziehen von Luft aufgrund der abströmenden Infusionsflüssigkeit in den Bereich stromab der Filtermembran verhindert. Damit ist ein Infusionsleitungssystem geschaffen, bei dem die Restmenge von Infusionsflüssigkeit, die nach dem Leerlaufen des Infusionsflüssigkeitsbehälters im Infusionsleitungssystem verbleibt, verringert ist.

Vorzugsweise sind die Kapillaren parallel und haben einen Durchmesser von maximal 3 µm, womit der Luftabscheider auch Partikel von mindestens 3 µm zurückhält.

Bei einer für kompakt bauende Infusionspumpen geeigneten Ausgestaltung des offenbarungsgemäßen Infusionsleitungssystems beträgt ein Abstand zwischen einem patientenseitigen Ende des Pumpenabschnitts und dem Einlass des Filtergehäuses höchstens 20 cm, vorzugsweise höchstens 10 cm.

Bei einer bevorzugten Weiterbildung des Infusionsleitungssystems ist an dem patientenseitigen Infusionsschlauch direkt stromab des Filters eine Schlauchklemme angeordnet, die als Schiebeklemme (Free Flow Protection Clamp FFPC) ausgebildet ist. Sie weist ein Langloch auf, in dem das patientenseitige Schlauchsegment aufgenommen ist. Das Langloch weist einen Schlitz (geschlossene Position) und einen gegenüber dem Schlitz (rundlich) erweiterten Bereich (offene Position) auf.

Eine besonders bevorzugte Ausgestaltung der Schiebeklemme hat eine vorbestimmte Farbe. Sie ist z.B. aus einem mit dieser Farbe eingefärbten Kunststoff gefertigt. Mit dieser Farbe ist einer von verschiedenen medizinischen Fachbereichen bzw. Anwendungsbereichen des Infusionsleitungssystems definiert, an dem die Schiebeklemme unverlierbar befestigt ist. Diese Farbe und damit den betroffenen medizinischen Fachbereich bzw. Anwendungsbereich kann der Bediener und/oder die Infusionspumpe über den Photodetektor des Klemmenmoduls erkennen.

Der Pumpenabschnitt, an dem das Fördermittel wirkt, kann integral mit dem behälterseitigen Infusionsschlauch gebildet sein, das heißt ein integraler (material- und eigenschaftsidentischer) Abschnitt des behälterseitigen Infusionsschlauchs sein.

Der Pumpenabschnitt des Infusionsleitungssystems, an dem das Fördermittel, - insbesondere die Peristaltik der Infusionspumpe - wirkt, kann ein extra Pumpensegment sein, das einen Teil des behälterseitigen Infusionsschlauches bildet, und das aus einem Material gefertigt ist, das von einem Material des übrigen behälterseitigen Infusionsschlauches abweicht. Das Material des Pumpensegments kann z.B. Silikon sein. Das Pumpensegment wird im Falle der peristaltischen Verdrängereinheit zur Förderung der Infusionsflüssigkeit durchgewalkt.

Dabei kann das Pumpensegment über zwei Kupplungsstücke mit dem übrigen behälterseitigen Infusionsschlauch fluiddicht verbunden sein. Damit ist das Pumpensegment über die beiden Kupplungsstücke in den behälterseitigen Infusionsschlauch eingesetzt.

Alternativ ist das Pumpensegment über ein behälterseitiges Kupplungsstück mit dem übrigen behälterseitigen Infusionsschlauch fluiddicht verbunden, während es über ein patientenseitiges Kupplungsstück direkt mit dem Einlass des Filters fluiddicht verbunden oder einstückig gebildet ist.

Bei einer vorrichtungstechnisch einfachen und besonders bevorzugten Weiterbildung mit dem extra Pumpensegment kann auch das patientenseitige Kupplungsstück komplett entfallen. Dann ist der Filter eingangsseitig mit Pumpabschnitt oder dem Pumpensegment und ausgangsseitig mit dem patientenseitigen Infusionsschlauch fluiddicht verbunden. Damit ist das Einlegen des Infusionsleitungssystems optimiert. Z.B. können einerseits das behälterseitige Kupplungsstück und andererseits der Filter als Positionierhilfe des Pumpensegments und damit des gesamten Infusionsleitungssystems dienen.

Gemäß einem Aspekt der besonders bevorzugten Weiterbildung kann das Pumpensegment über ein behälterseitiges Kupplungsstück mit dem behälterseitigen Infusionsschlauch (fluiddicht) verbunden sein.

Gemäß einem weiteren Aspekt der besonders bevorzugten Weiterbildung kann das Pumpensegment über den Filter oder das Filtergehäuse mit dem patientenseitigen Infusionsschlauch (fluiddicht) verbunden sein.

Gemäß der besonders bevorzugten Weiterbildung ist es in anderen Worten bevorzugt, wenn der Filter oder das Filtergehäuse ein patientenseitiges Kupplungsstück bilden bzw. dieses ersetzen. Es ist entsprechend in vorteilhafter Weise kein zu dem Filter oder Filtergehäuse zusätzliches patientenseitiges Kupplungsstück an einem axialen Ende des Pumpensegments vorgesehen.

Bevorzugt ist entsprechend gemäß der besonders bevorzugten Weiterbildung an dem offenbarungsgemäßen Infusionsleitungssystem lediglich ein im Wesentlichen bzw. zumindest abschnittsweise rotationssymmetrisches Kupplungsstück vorgesehen, das heißt bevorzugt sind nicht zwei derartige im Wesentlichen bzw. zumindest abschnittsweise rotationssymmetrische Kupplungsstücke vorgesehen.

Wenn das Infusionsleitungssystem ohne von dem Filter separatem patientenseitigen Kupplungsstück ausgebildet ist, also in anderen Worten der Filter oder das Filtergehäuse das patientenseitige Kupplungsstück bilden, ist bevorzugt die offenbarungsgemäße Infusionspumpe vorrichtungstechnisch an diese bevorzugte Ausführungsform des Infusionsleitungssystems angepasst.

Die offenbarungsgemäße Infusionspumpe kann entsprechend gemäß der besonders bevorzugten Weiterbildung nur eine Kupplungsstückaufnahme (für das behälterseitige Kupplungsstück), sowie eine Filteraufnahme für das Filtergehäuse oder den Filter aufweisen. Entsprechend ist bevorzugt keine separate Kupplungsstückaufnahme für ein patientenseitiges Kupplungsstück vorgesehen, da bevorzugt kein separates patientenseitiges Kupplungsstück in dem Infusionsleitungssystem vorgesehen ist, sondern durch das Filtergehäuse bzw. den Filter gebildet wird.

Bevorzugt weist die Infusionspumpe entsprechend lediglich eine Kupplungsstückaufnahme zur Aufnahme eines im Wesentlichen bzw. zumindest abschnittsweise rotationssymmetrischen Kupplungsstücks, beispielsweise zumindest abschnittsweise zylindrischen Kupplungsstücks, auf. In anderen Worten ist bevorzugt keine zweite Kupplungsstückaufnahme zur Aufnahme eines im Wesentlichen bzw. zumindest abschnittsweise rotationssymmetrischen Kupplungsstücks, beispielsweise zumindest abschnittsweise zylindrischen Kupplungsstücks, vorgesehen, sondern die offenbarungsgemäße Filteraufnahme.

Offenbart ist selbstverständlich auch ein Infusionssystem mit einer offenbarungsgemäßen Infusionspumpe und mit einem offenbarungsgemäßen Infusionsleitungssystem. Auch dieses löst die beiden gestellten Aufgaben.

Bei einer Weiterbildung des offenbarungsgemäßen Infusionssystems hat die Schlauchaufnahme die durchgehende Mittelachse, entlang der das (gerade ausgerichtete) Infusionsleitungssystem teilweise einlegbar ist. Dabei ist der Filteraufnahmeraum von der Mittelachse durchdrungen, und das Filtergehäuse hat den Einlass und den Auslass, die beide konzentrisch zur Mittelachse sind.

Auch die Verwendung eines offenbarungsgemäßen Infusionsleitungssystems in einer offenbarungsgemäßen Infusionspumpe löst die beiden gestellten Aufgaben.

### Kurzbeschreibung der Figuren

Figur 1 zeigt ein Infusionsleitungssystem gemäß einem ersten Ausführungsbeispiel der vorliegenden Offenbarung;
Figur 2 zeigt einen Filter der Infusionsleitungssysteme aus Figur 1 und aus Figur 6;
Figur 3 zeigt den Filter aus Figur 2 in einer Explosionsdarstellung;
Figur 4 zeigt eine Infusionspumpe gemäß einem Ausführungsbeispiel der vorliegenden Offenbarung in einer schematischen Frontansicht;
Figur 5 zeigt eine Schiebeklemme der Infusionsleitungssysteme aus Figur 1 und aus Figur 6; und
Figur 6 zeigt einen zentralen Ausschnitt eines Infusionsleitungssystems gemäß einem zweiten Ausführungsbeispiel der vorliegenden Offenbarung.

### Beschreibung der Ausführungsbeispiele

Nachstehend werden zwei Ausführungsbeispiele eines Infusionssystems gemäß der vorliegenden Offenbarung beschrieben. Dazu werden Ausführungsbeispiele des Infusionsleitungssystems und der Infusionspumpe auf der Basis der zugehörigen Figuren beschrieben.

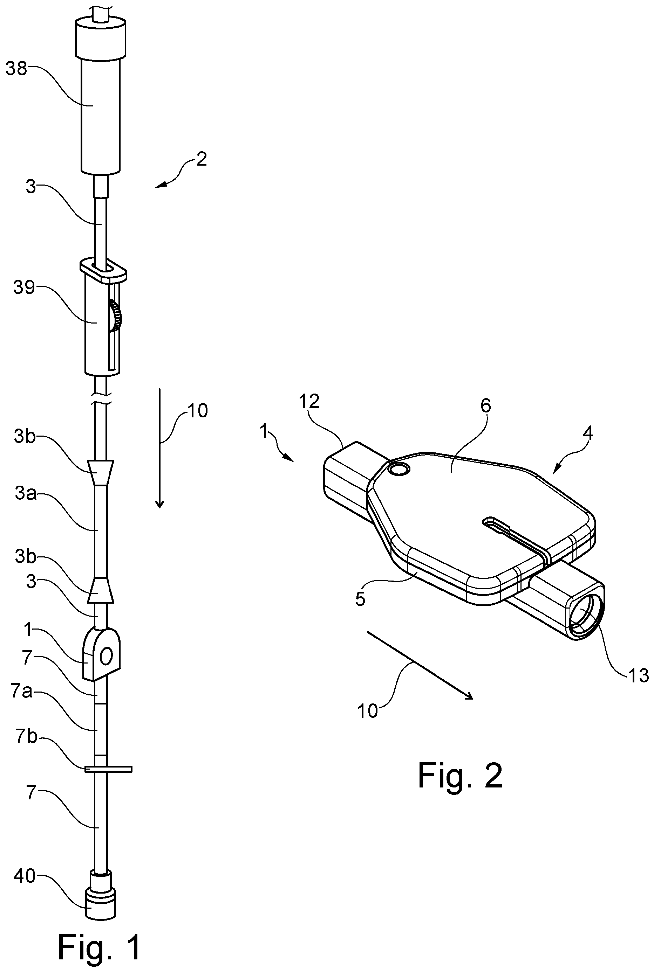

Figur 1 zeigt ein Infusionsleitungssystem 2 gemäß einem ersten Ausführungsbeispiel der vorliegenden Offenbarung mit einem darin eingesetzten Filter 1. Stromauf des Filters 1 ist ein (nicht zum Infusionsleitungssystem 2 gehörender) Behälter zur Aufnahme/Lagerung der Infusionsflüssigkeit angeordnet. Daran ist eine Tropfkammer 38 des Infusionsleitungssystems 2 angeschlossen, welche eine erste Vorrichtung zur Luftabscheidung darstellt. An die Tropfkammer 38 ist ein behälterseitiger Infusionsschlauch 3 mit einer Schlauchklemme 39 zur Regelung der Strömung angeordnet. In den patientenseitigen Infusionsschlauch 3 ist ein Pumpensegment 3a über Kupplungsstücke 3b eingesetzt. Das Pumpensegment 3a hat eine gegenüber dem übrigen behälterseitigen Infusionsschlauch 3 erhöhte Qualität, da hier eine weiter unten beschriebene peristaltische Verdrängereinheit der Infusionspumpe wirkt.

Stromab des Filters 1 ist über einen patientenseitigen Infusionsschlauch 7 ein (Patienten-)Anschluss 40 angeschlossen, welcher mit einer Injektionsstelle eines Patienten oder einer weiteren Infusionsleitung fluidisch verbunden werden kann.

In dem patientenseitigen Infusionsschlauch 7 ist ein Sensorabschnitt 7a angeordnet oder gebildet. Der Sensorabschnitt 7a kann gegenüber dem übrigen behälterseitigen Infusionsschlauch 3 unverändert sein und wird in einen weiter unten beschriebenen Luftsensor der Infusionspumpe eingesetzt.

An dem patientenseitigen Infusionsschlauch 7 stromab des Sensorabschnitts 7a und benachbart dazu ist eine Schiebeklemme 7b unverlierbar angeordnet. Die Schiebeklemme 7b dient zum Absperren und zum Freigeben des Infusionsleitungssystems 2 und ist einstückig aus einem Kunststoff spritzgegossen, der mit einer vorbestimmten Farbe eingefärbt ist. Damit wird eine Farberkennung realisiert. Die Farbe bedeutet einen bestimmten medizinischen Fachbereich für das Infusionsleitungssystem 2.

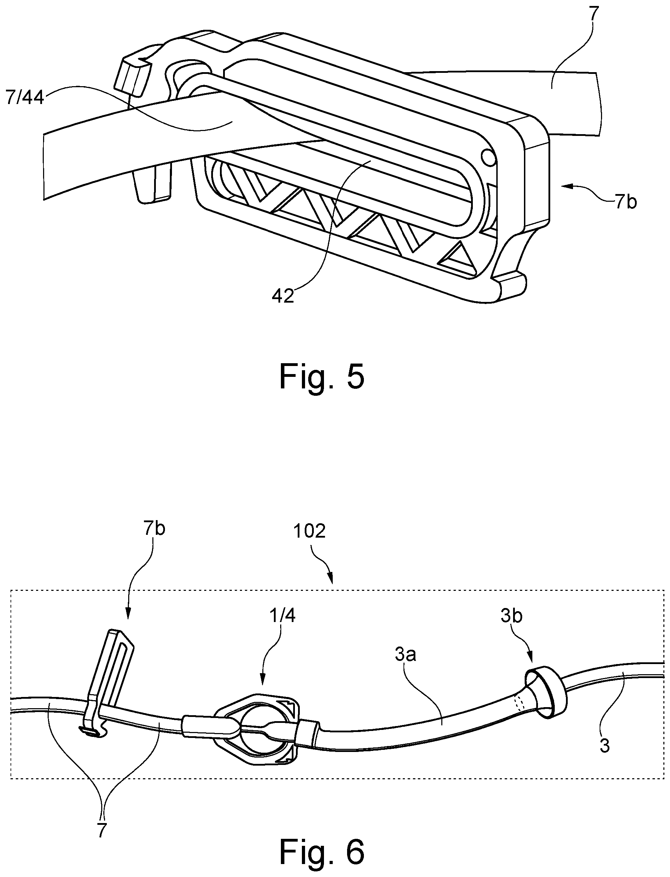

Der körperliche Aufbau der Schiebeklemme 7b wird mit Bezug zu Figur 5 genauer erläutert.

Das Pumpensegment 3a mit seinen Kupplungsstücken 3b einerseits und der Sensorabschnitt 7a andererseits sind unmittelbar benachbart zum Filter 1 angeordnet, es sind also abgesehen von Schlauchabschnitten 3, 7 keine weiteren Vorrichtungen oder Teile oder Ventile oder Kupplungen oder Hähne dazwischen angeordnet. Beim gezeigten Ausführungsbeispiel beträgt der Abstand des Filters 1 zum Kupplungsstück 3b des Pumpensegments 3a einerseits und der Abstand des Filters 1 zum Sensorabschnitt 7a andererseits nur maximal 5 cm.

Somit strömt die Infusionsflüssigkeit gemäß der Strömungsrichtung 10 aus dem Behälter durch die Tropfkammer 38, die Schlauchklemme 39, das Pumpensegment 3a, den Filter 1, den Sensorabschnitt 7a zum Anschluss 40. Die vorstehend beschriebene turbulenzfreie Durchströmung des Filters 1 ermöglicht es, den Filter 1 im Nahbereich des (Patienten-)Anschlusses 40 anzuordnen.

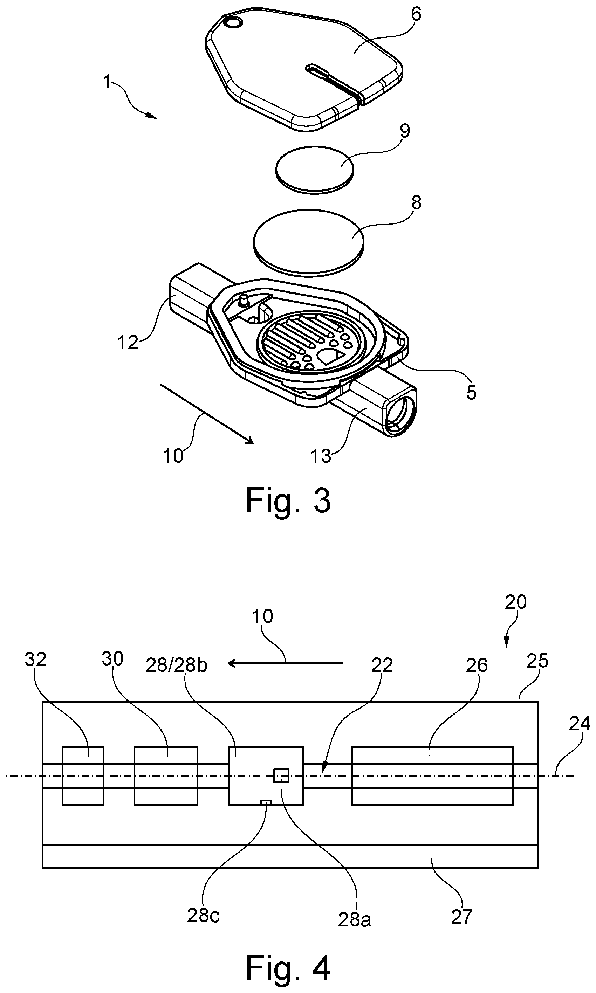

Figur 2 zeigt eine perspektivische Ansicht und Figur 3 eine Explosionsdarstellung des Filters/Luftabscheiders 1 des vorstehend beschriebenen Infusionsleitungssystems 2. Der Filter 1 ist in den die Infusionsflüssigkeit führenden Infusionsschlauch zwischengeschaltet. Er dient der Luftabscheidung und darüberhinausgehend auch der Abscheidung von Partikeln. Daher ist der Filter 1 genauer gesagt ein Luftabscheider und Partikelfilter.

Wie in Figur 2 gezeigt, weist der Filter 1 ein Filtergehäuse 4 mit einem Gehäuseunterteil 5 und einem form- und/oder kraftschlüssig mit dem Gehäuseunterteil 5 verbundenen Gehäusedeckel 6 auf. Im Inneren des Filtergehäuses 4, d.h. im Bereich einer zwischen dem Gehäuseunterteil 5 und dem Gehäusedeckel 6 ausgebildeten Kavität (Hohlraum), sind, wie in Figur 3 zu erkennen, eine hydrophile (Fluid-) Filtermembran 8 und ein Entlüftungsfilter 9 angeordnet. Die Filtermembran 8 dient dabei als Luftbarriere und der Entlüftungsfilter 9 ermöglicht den Transport von Luft, welche sich im Bereich der Kavität sammelt, zur Außenseite des Filtergehäuses 4.

Einstückig mit dem Gehäuseunterteil 5 sind ein stutzenartiger Einlass 12 und ein stutzenartiger Auslass 13 gebildet. Damit ist der Filter 1 eingangsseitig an den behälterseitigen Infusionsschlauch 3 und ausgangsseitig an den patientenseitigen Infusionsschlauch 7 angeschlossen.

Figur 4 zeigt eine Infusionspumpe 20 gemäß einem Ausführungsbeispiel der vorliegenden Offenbarung in einer schematischen Frontansicht, wobei eine Frontklappe 27 aufgeklappt ist. Damit ist eine Schlauchaufnahme 22 sichtbar und zugänglich, die eine durchgehende Mittelachse 24 hat. Entlang dieser Mittelachse 24 wird das Infusionsleitungssystem 2 aus Figur 1 teilweise in die Schlauchaufnahme 22 eingelegt. Dabei wird das Filtergehäuse 4 des Filters 1 in eine Filteraufnahme 28 mittels der Schnappverbindung 28a eingeclipst, die (entgegen der schematischen Darstellung) den Umriss und die Form des konkaven Gehäuseunterteils 5 des Filtergehäuses 4 einschließlich des Einlasses 12 und des Auslasses 13 in Form einer konkaven Aussparung 28b aufweist.

Die Anwesenheit des Gehäuseunterteils 5 und damit die Anwesenheit des Filtergehäuses 4 und damit die Anwesenheit des Filters 1 wird mittels eines Sensors 28c überwacht.

Entlang der Mittelachse 24 stromab der Filteraufnahme 28 ist ein Luftsensor 30 vorgesehen. Damit ist offenbarungsgemäß sichergestellt, dass der in die Filteraufnahme 28 eingesetzte Filter 1 (siehe Figuren 1 und 2) an letztmöglicher Stelle eventuelle Luftblasen aus der Infusionsflüssigkeit abscheidet, bevor die (Erfolgs-) Kontrolle der Luftabscheidung erfolgt.

Die (in Figur 1 gezeigte) Schiebeklemme 7b wird in ein Klemmenmodul 32 eingesteckt. Da die Schiebeklemme 7b mit einer vorbestimmten Farbe eingefärbt ist, kann die Schiebeklemme 7b Licht an einen (nicht gezeigten) Photodetektor des Klemmenmoduls 32 zurücksenden. Dadurch kann die Infusionspumpe 20 den medizinischen Fachbereich erkennen und z.B. auf einem Display nur die zugehörigen Funktionen anzeigen.

Stromauf der Filteraufnahme 28 ist eine peristaltische Verdrängereinheit 26 angeordnet, die beim gezeigten Ausführungsbeispiel translatorisch ausgeführt ist. Dabei oszilliert eine Vielzahl einzelner nicht näher gezeigter schieberartiger Verdrängungskörper radial senkrecht zur Zeichenebene und quetscht dabei das (in Figur 1 gezeigte) Pumpensegment 3a des behälterseitigen Infusionsschlauches 3 derart, dass die Infusionsflüssigkeit in Strömungsrichtung 10 durch das gesamte (in Figur 1 gezeigte) Infusionsleitungssystem 2 gefördert wird.

An den beiden axialen Enden der peristaltischen Verdrängereinheit 26 können gemäß einem Ausführungsbeispiel Kupplungsstückaufnahmen vorgesehen sein, insbesondere für die Kupplungsstücke 3b des Infusionsleitungssystems 2.

Figur 5 zeigt die Schiebeklemme 7b des Infusionsleitungssystems 2 aus Figur 1 und des Infusionsleitungssystems 102 aus Figur 6. Sie hat ein Langloch, das aus einem Schlitz 42 und einem erweiterten Bereich 44 zusammengesetzt ist. In der in Figur 5 gezeigten Position ist der patientenseitige Infusionsschlauch 7 im erweiterten Bereich 44 aufgenommen, so dass der patientenseitige Infusionsschlauch 7 geöffnet ist.

Figur 6 zeigt einen zentralen Ausschnitt eines zweiten Ausführungsbeispiels eines Infusionsleitungssystems 102, bei dem gegenüber dem Ausführungsbeispiel aus Figur 1 vorrichtungstechnisch einfach das patientenseitige Kupplungsstück 3b komplett entfallen ist. Der Filter 1 bzw. das Filtergehäuse 4 ist eingangsseitig direkt mit dem Pumpensegment 3a und ausgangsseitig mit dem patientenseitigen Infusionsschlauch 7 fluiddicht verbunden. Als Positionierhilfe des Infusionsleitungssystems 102 bzw. seines Pumpensegments 3a dient dann einerseits das behälterseitige Kupplungsstück 3b und andererseits der Filter 1 bzw. das Filtergehäuse 4.

Zurückverweisend auf Fig. 4 müsste eine offenbarungsgemäße Infusionspumpe 20, um für das Infusionsleitungssystem 102 angepasst bzw. geeignet zu sein, derart modifiziert werden, dass lediglich an dem in Strömungsrichtung 10 stromaufwärtigen Ende der Verdrängereinheit 26 eine behälterseitige Kupplungsstückaufnahme vorgesehen ist, während hingegen die stromabwärtige patientenseitige Kupplungsstückaufnahme entfallen würde. Entsprechend würde die Filteraufnahme 28 in Fig. 4 nach rechts verrückt werden, und die Filteraufnahme 28 würde dann im Wesentlichen eine patientenseitige Kupplungsstückaufnahme bilden, so dass eine separate patientenseitige Kupplungsstückaufnahme entfallen könnte.

Abweichend von den in den Figuren 1 und 6 gezeigten Pumpensegmenten 3a mit den beiden Kupplungsstücken 3b oder mit dem einen Kupplungsstück 3b könnten diese Teile auch entfallen, so dass der behälterseitige Schlauch 3 ohne Materialänderung in die (in Figur 4 gezeigte) peristaltische Verdrängereinheit 26 eingesetzt wird.

Offenbart ist damit auch ein Infusionssystem mit der Infusionspumpe 20 und mit dem Infusionsleitungssystem 2; 102. In dem Infusionsleitungssystem 2; 102 ist der Filter 1 zwischengeschaltet bzw. eingesetzt, der eine Filtermembran 8 vorzugsweise mit einer Vielzahl von parallelen Kapillaren aufweist. Der Filter 1 ist an einer Stelle in dem Infusionsleitungssystem 2; 102 angeordnet, die nach dessen Einsetzen in die Infusionspumpe 20 direkt stromauf von einem Luftsensor 30 der Infusionspumpe 20 angeordnet ist.

### Bezugszeichenliste:

- 1: Filter
- 2; 102: Infusionsleitungssystem
- 3: behälterseitiger Infusionsschlauch
- 3a: Pumpensegment
- 3b: Kupplungsstück
- 4: Filtergehäuse
- 5: Gehäuseunterteil
- 6: Gehäusedeckel
- 7: patientenseitiger Infusionsschlauch
- 7a: Sensorabschnitt
- 7b: Schiebeklemme
- 8: Filtermembran
- 9: Entlüftungsfilter
- 10: Strömungsrichtung
- 12: Einlass
- 13: Auslass
- 20: Infusionspumpe
- 22: Schlauchaufnahme
- 24: Mittelachse
- 25: Hauptkörper
- 26: peristaltische Verdrängereinheit
- 27: Frontklappe
- 28: Filteraufnahme
- 28a: Schnappverbindung
- 28b: Aussparung
- 28c: Sensor
- 30: Luftsensor
- 32: Klemmenmodul
- 38: Tropfkammer
- 39: Schlauchklemme
- 40: (Patienten-)Anschluss
- 42: Schlitz
- 44: erweiterter Bereich

## Patentansprüche

1. Infusionspumpe (20) mit einer Schlauchaufnahme (22) zum teilweisen Einlegen eines Infusionsleitungssystems (2; 102), wobei in der Schlauchaufnahme (22) ein Fördermittel, vorzugsweise eine peristaltische Verdrängereinheit (26), angeordnet ist, wobei in der Schlauchaufnahme (22) stromabwärts des Fördermittels eine Filteraufnahme (28) mit einem Filteraufnahmeraum für ein Filtergehäuse (4) oder eines Filtergehäuses (4) vorgesehen ist.

2. Infusionspumpe (20) nach Anspruch 1, wobei in der Schlauchaufnahme (22) stromabwärts der Filteraufnahme (28) ein Luftsensor (30) angeordnet ist, mit dem Luft in dem Infusionsleitungssystem (2; 102) erkennbar ist.

3. Infusionspumpe (20) nach Anspruch 1 oder 2, wobei die Filteraufnahme (28) eine Clips- oder Schnappverbindung (28a) für das Filtergehäuse (4) aufweist.

4. Infusionspumpe (20) nach einem der vorhergehenden Ansprüche, mit einem Hauptkörper (25), an den eine Frontklappe (27) angelenkt ist, wobei die Schlauchaufnahme (24) zumindest abschnittsweise durch die Frontklappe (27) abdeckbar ist, und wobei der Filteraufnahmeraum durch die geschlossene Frontklappe (27) abgedeckt und begrenzt ist, womit vorzugsweise das Filtergehäuse (4) in dem Filteraufnahmeraum festlegbar ist.

5. Infusionspumpe (20) nach einem der vorhergehenden Ansprüche, wobei der Filteraufnahmeraum von einer Aussparung (28b) oder Mulde begrenzt oder gebildet ist.

6. Infusionspumpe (20) nach den Ansprüchen 4 und 5, wobei die Aussparung (28b) oder Mulde in der Frontklappe (27) oder im Hauptkörper (25) gebildet ist.

7. Infusionspumpe (20) nach einem der vorhergehenden Ansprüche mit einem Sensor (28c) zur Erfassung des Filtergehäuses (4).

8. Infusionsleitungssystem (2; 102), das zum teilweisen Einlegen in eine Infusionspumpe (20) nach einem der vorhergehenden Ansprüche eingerichtet und ausgelegt ist, wobei das Infusionsleitungssystem (2; 102) einen Filter (1) mit einem Filtergehäuse (4) aufweist, das über einen Einlass (12) mit einem behälterseitigen Infusionsschlauch (3) und über einen Auslass (13) mit einem patientenseitigen Infusionsschlauch (7) verbunden ist, wobei in dem Filtergehäuse (4) eine, vorzugsweise hydrophile, Filtermembran (8) angeordnet ist, die vorzugsweise eine Vielzahl von hydrophilen Kapillaren aufweist, wobei an dem behälterseitigen Infusionsschlauch (3) ein Pumpenabschnitt angeordnet oder gebildet ist, der in Wirkverbindung mit einem Fördermittel, vorzugsweise einer peristaltischen Verdrängereinheit (26), der Infusionspumpe (20) bringbar ist.

9. Infusionsleitungssystem (2; 102) nach Anspruch 8, wobei an dem patientenseitigen Infusionsschlauch (7) ein Sensorabschnitt (7a) angeordnet oder gebildet ist, der in Wirkverbindung mit einem Luftsensor (30) der Infusionspumpe (20) bringbar ist.

10. Infusionsleitungssystem (2; 102) nach Anspruch 8 oder 9, wobei ein Abstand zwischen dem Pumpenabschnitt und dem Einlass (12) des Filtergehäuses (4) höchstens 20 cm, vorzugsweise höchstens 10 cm beträgt.

11. Infusionsleitungssystem (2; 102) nach einem der Ansprüche 8 bis 10, wobei an dem patientenseitigen Infusionsschlauch (7) stromab des Pumpenabschnitts und/oder des Filters (1) eine Schlauchklemme angeordnet ist, die insbesondere als Schiebeklemme (7b) ausgebildet ist, die ein Langloch aufweist, in dem das patientenseitige Schlauchsegment (7) aufgenommen ist, wobei das Langloch von einem Schlitz (42) und einen gegenüber dem Schlitz (42) erweiterten Bereich (44) gebildet ist.

12. Infusionsleitungssystem (2; 102) nach einem der Ansprüche 8 bis 11, wobei der Pumpenabschnitt ein extra Pumpensegment (3a) ist, das einen Teil des behälterseitigen Infusionsschlauches (3) bildet, und das aus einem Material gefertigt ist, das von dem Material des übrigen behälterseitigen Infusionsschlauches (3) abweicht.

13. Infusionsleitungssystem (102) nach Anspruch 12, wobei der Filter (1) oder das Filtergehäuse (4) unmittelbar an den Pumpabschnitt angeordnet oder an dem extra Pumpensegment (3a) befestigt ist.

14. Infusionssystem mit einer Infusionspumpe (20) nach einem der Ansprüche 1 bis 7 und mit einem Infusionsleitungssystem (2; 102) nach einem der Ansprüche 8 bis 13, wobei vorzugsweise die Schlauchaufnahme (22) eine durchgehende Mittelachse (24) hat, entlang der das Infusionsleitungssystem (2; 102) einlegbar oder eingelegt ist, und wobei vorzugsweise der Filteraufnahmeraum von der Mittelachse (24) durchdrungen ist und das Filtergehäuse (4) einen Einlass (12) und einen Auslass (13) hat, die beide konzentrisch zur Mittelachse (24) sind.

15. Verwendung eines Infusionsleitungssystems (2; 102) nach einem der Ansprüche 8 bis 13 mit einer Infusionspumpe (20) nach einem der Ansprüche 1 bis 7.
